(19) Europäisches Patentamt
European Patent Office
Office européen des brevets

(11) Veröffentlichungsnummer: **0 063 271**
**B2**

(12) **NEUE EUROPÄISCHE PATENTSCHRIFT**

(45) Veröffentlichungstag der neuen Patentschrift: **03.10.90**

(51) Int. Cl.$^5$: **C 07 C 303/06**

(21) Anmeldenummer: **82102730.7**

(22) Anmeldetag: **31.03.82**

# (54) Verfahren zur Herstellung von aromatischen Amino-sulfonsäuren.

(30) Priorität: **11.04.81 DE 3114829**

(43) Veröffentlichungstag der Anmeldung:
**27.10.82 Patentblatt 82/43**

(45) Bekanntmachung des Hinweises auf die Patenterteilung:
**12.12.84 Patentblatt 84/50**

(45) Bekanntmachung des Hinweises auf die Entscheidung über den Einspruch:
**03.10.90 Patentblatt 90/40**

(84) Benannte Vertragsstaaten:
**BE CH DE FR GB IT LI NL**

(56) Entgegenhaltungen:
**DE-A-2 439 297**
**US-A-2 471 400**

**HOUBEN WEYL "Methoden der organischen Chemie" 4. Auflage, Band IX, 1955, GEORG THIEME VERLAG, Stuttgart**
**Ind. Eng. Chem.,35 (1943), S. 321-323**
**Ind. Eng. Chem., 42 (1950), S. 1746ff**
**FIAT N87 Frames, 4689-93 (1933), S. 1-5**

(73) Patentinhaber: **BAYER AG**
**D-5090 Leverkusen 1 Bayerwerk (DE)**

(72) Erfinder: **Emde, Herbert, Dr.**
**Andreas-Gryphiusstrasse 7**
**D-5000 Koeln 80 (DE)**
Erfinder: **Blank, Heinz Ulrich, Dr.**
**Am Geusfelde 35**
**D-5068 Odenthal (DE)**
Erfinder: **Schnegg, Peter, Dr.**
**Heidbergerstrasse 44**
**D-5068 Odenthal (DE)**

**EP 0 063 271 B2**

**Beschreibung**

Die vorliegende Erfindung betrifft ein Verfahren zur Herstellung von Amino-arylsulfonsäuren aus einem Arylamin und Schwefelsäure nach dem sogenannten Backverfahren.

Es ist bereits bekannt, daß man aromatische Aminosulfonsäuren aus einem Arylamin und Schwefelsäure bei erhöhter Temperatur erhält (Helv. Chem. Acta *15*, 1372 (1932)). In diesem sogenannten Backprozeß wird in einer ersten Stufe das zugehörige Arylammoniumhydrogensulfat gebildet, welches dann bei erhöhter Temperatur in Substanz oder in einem inerten Lösungsmittel unter Wasseraustritt zur entsprechenden Amino-sulfonsäure umgesetzt wird. Im allgemeinen wird zur Steigerung der Reaktionsgeschwindigkeit und zur Vervollständigung der gewünschten Reaktion das entstehende Wasser allein oder bei Verwendung eines Lösungsmittels mit diesem zusammen möglichst rasch und vollständig, meist unter Anlegen von Vakuum (BIOS 11536, S. 175, S. 182, 188 und Ind. Eng. Chem. *42*, 1746 (1950)) aus dem Reaktionsgemisch entfernt. Bei dieser Verfahrensweise werden Aminoaryl-disulfonsäuren sowie isomere Aminoarylsulfonsäuren als unerwünschte Nebenprodukte sowie häufig dunkelgefärbte Produkte erhalten (Russ. Patent 667550). Zudem sind lange Reaktionzeiten bei mäßigen Ausbeuten erforderlich (Ind. Eng. Chem., loc. cit.).

Es wurde nun ein Verfahren zur Herstellung von Aminoarylsulfonsäuren durch Umsetzung von Arylaminen mit Schwefelsäure in Gegenwart eines Lösungsmittels nach dem sogenannten Backverfahren bei einer Temperatur von 140°C bis 280°C gefunden, das dadurch gekennzeichnet ist, daß bei der Umsetzung im Reaktionsabschnitt, in dem 70 bis 100% des gesamten Reaktionsumsatzes erzielt werden, Wasser nur in einer solchen Menge entfernt wird, daß stets 0,02 bis 2 Mol Wasser pro Mol ursprünglich in das Reaktionsgemisch eingebrachten Amins im Reaktionsansatz verbleibt, und die Umsetzung im Druckbereich zwischen dem Partialdampfdruck des Lösungsmittels unter den Bedingungen des Verfahrens und dem maximal möglichen Druck des Reaktionssystems durchgeführt wird, wobei man unter einem Druck von 1,1 bis 50 bar arbeitet.

Im erfindungsgemäßen Verfahren können Arylamine der allgemeinen Formel (I)

$$Ar^1{-}N(R^1)(R^2) \qquad (I)$$

eingesetzt werden, in der

$R^1$ und $R^2$ unabhängig voneinander Wasserstoff, Alkyl, Aralkyl oder Aryl bedeuten oder beide gemeinsam mit dem N-Atom, das sie substituieren, einen Stickstoffheterocyclus bilden und

$Ar^1$ das gegebenenfalls substituierte Benzol-, Naphthalin-, Anthracen-, Naphthochinon- oder Anthrachinongerüst oder das Gerüst eines aromatischen Heterocyclus darstellt.

Als Alkyl sei beispielsweise ein solches mit 1 bis 8, bevorzugt 1 bis 4, besonders bevorzugt 1 bis 2 C-Atomen genannt, wie Methyl, Ethyl, Propyl, Isopropyl, Butyl, Isobutyl, Hexyl oder Octyl.

Als Aralkyl sei beispielsweise Benzyl, 1-Phenyl-ethyl, 2-Phenyl-ethyl, Naphthylmethyl, Naphthylethyl, Anthrylmethyl oder Anthrylethyl, bevorzugt Benzyl, genannt.

Als Aryl sei beispielsweise Phenyl, substituiertes Phenyl, Naphthyl oder Diphenyl, bevorzugt Phenyl, genannt.

Für den Fall, daß $R^1$ und $R^2$ gemeinsam mit dem N-Atom, das sie substituieren, einen Stickstoffheterocyclus bilden, sei ein solcher mit beispielsweise 4 bis 8, bevorzugt 5 oder 6 Ringgliedern genannt, wie Pyrrolidin oder Piperidin.

Das substituierte Benzol-, Naphthalin-, Anthracen-, Naphthochinon- oder Anthrachinongerüst kann neben der Aminogruppe $—NR^1R^2$ beispielsweise bis zu 3 weitere Substituenten, bevorzugt bis zu 2 Substituenten aufweisen, wobei die Substituenten so angeordnet sein sollen, daß mindestens eine ortho- oder para-Stellung unsubstituiert ist. Als Substituenten seien beispielsweise genannt: Alkyl im Rahmen des o.g. Bedeutungsumfanges, Trifluormethyl, Perfluorethyl; weitere Substituenten sind beispielsweise Phenyl, Alkoxy mit 1 bis 4, bevorzugt 1 bis 2 C-Atomen, beispielsweise Methoxy, Ethoxy, Propoxy, Isopropyloxy, Butoxy oder Isobutyloxy, Alkylthio, wie die Thioanaloga der genannten Alkoxygruppen, Halogen, wie Fluor, Chlor oder Brom, weiterhin Hydroxy, Nitro, gegebenenfalls substituiertes Amino, $SO_3H$ oder Carboxyl sowie Alkylsulfonyl oder Arylsulfonyl, wie Methyl-, Ethyl- oder Phenylsulfonyl. Als Substituenten seien bevorzugt Methyl, Ethyl, Phenyl, Methoxy, Ethoxy, Halogen, Hydroxyl, Nitro oder Amino genannt. Ganz besonders bevorzugt als Substituenten seien Methyl, Chlor, Brom, Fluor, Methoxy oder Ethoxy genannt.

Bevorzugte, erfindungsgemäß einsetzbare Arylamine sind solche der Formel (II)

$$Ar^1{-}N(R^3)(R^4) \qquad (II),$$

in der

$Ar^1$ die genannte Bedeutung hat und

$R^3$ und $R^4$ unabhängig voneinander für Wasserstoff oder Alkyl stehen.

Besonders bevorzugte Arylamine für das erfindungsgemäße Verfahren sind solche der Formel (III)

$$Ar^1—NH_2 \qquad (III),$$

in der

$Ar^1$ die genannte Bedeutung hat.

Weitere bevorzugte Arylamine für das erfindungsgemäße Verfahren sind solche der Formel (IV)

$R^1$
/
$Ar^2$—N (IV),
\
$R^2$

in der

$R^1$ und $R^2$ die obengenannte Bedeutung haben und

$Ar^2$ das Benzol- oder Naphthalingerüst darstellt.

Weitere besonders bevorzugte Arylamine für das erfindungsgemäße Verfahren sind solche der Formel (V)

$R^3$
/
$Ar^2$—N (V),
\
$R^4$

in der

$R^3$, $R^4$ und $Ar^2$ die genannte Bedeutung besitzen.

In ganz besonders bevorzugter Weise werden Arylamine der Formel (VI)

$$Ar^2—NH_2 \qquad (VI)$$

eingesetzt, in der

$Ar^2$ die genannte Bedeutung besitzt und insbesondere das Benzolgerüst darstellt.

Beispiele für im erfindungsgemäßen Verfahren einsetzbare Arylamine sind Anilin, o-Toluidin, m-Toluidin, p-Toluidin, 2,4-Dimethyl-anilin, 2,3-Dimethyl-anilin, 2,6-Dimethylanilin, 2,5-Dimethyl-anilin, N-Methyl-anilin, N-Ethyl-anilin, N,N-Dimethyl-anilin, Diphenylamin, p-Chloranilin, 2,4-Dichlor-anilin, o-Chloranilin, 2,3-Dichloranilin, 3,5-Dichloranilin, 2,5-Dichloranilin, 2,6-Dichloranilin, m-Chloranilin, 2-Amino-6-chlor-toluol, 2-Amino-5-chlor-toluol, 2-Amino-4-chlor-toluol, 2-Methoxy-5-methyl-anilin, p-Methoxy-anilin, o-Nitroanilin, m-Nitroanilin, p-Nitroanilin, α-Naphthylamin, p-Phenylendiamin, m-Phenylendiamin, Amino-diphenyl, p-Nitrodiphenylamin, 2-Methoxy-4-nitro-anilin, 1-Amino-2-ethoxy-naphthalin, 1-Amino-2-hydroxy-naphthalin, 1-Amino-8-hydroxy-naphthalin, 1-Amino-5-hydroxy-naphthalin, 1,8-Diamino-naphthalin, 1,5-Diamino-naphthalin, 2-Amino-3-hydroxy-naphthalin, 2-Amino-pyridin, 3-Chlor-4-methoxy-anilin, 2-Aminobenzoesäure, p-Ethoxy-anilin, 3,4-Dichloranilin, o-Fluoranilin, m-Fluoranilin, p-Fluoranilin, 2-Amino-3-chlortoluol, 3-Amino-2-chlortoluol, 5-Amino-2-chlortoluol, 3-Amino-5-chlortoluol, 3-Amino-4-chlortoluo, 4-Amino-3-chlortoluol, 4-Amino-2-chlortoluol, 5-Methoxy-2-methylanilin, 2,3-, 2,4-, 2,5-, 2,6-, 3,4-, 3,5-Dimethoxy- und Diethoxyanilin, o-Methoxy-anilin, m-Methoxy-anilin, N-Acetyl-p-Phenylendiamin, 2-Chlor-4-methoxy-anilin, 2-Chlor-3-methoxy-anilin, 4-Chlor-3-methoxy-anilin, 5-Chlor-3-methoxy-anilin, 2-Chlor-5-methoxy-anilin, 3-Chlor-2-methoxy-anilin, 4-Chlor-2-methoxy-anilin, 5-Chlor-2-methoxy-anilin, 2-Chlor-6-methoxy-anilin sowie die analogen Chlor-ethoxy-aniline, 3-Amino-6-chlor-benzoesäure, o-Trifluormethyl-anilin, m-Trifluormethyl-anilin, p-Trifluormethyl-anilin, Aminoanthrachinone wie z.B. 1-Aminoanthrachinon oder 1,5-Diaminoantrachinon, Benzidin und Dehydrothiotoluidin.

Das Arylamin wird mit Schwefelsäure im Verhältnis von 0,5 bis 1,1, bevorzugt 0,90 bis 1,05, besonders bevorzugt 0,95 bis 1,02, Mol Schwefelsäure pro Mol Amin, gegebenenfalls in Gegenwart von zusätzlichem Alkalimetallhydrogensulfat, umgesetzt. In ganz besonders bevorzugter Weise werden Schwefelsäure und Amin im Molverhältnis von etwa 1:1 umgesetzt. Man kann aber auch die entsprechenden Arylammoniumhydrogensulfate in fester Form, als Suspension oder als Schmelze für das erfindungsgemäße Verfahren einsetzen.

Die Schwefelsäure kann als konzentrierte Schwefelsäure (sog. Monohydrat) oder als verdünnte Schwefelsäure eingesetzt werden. Beispielsweise sei ein $H_2SO_4$-Gehalt von 70 bis 100 Gew.-% genannt. Es ist jedoch auch möglich, eine Schwefelsäure einzusetzen, die freies Schwefeltrioxid gelöst enthält, beispielsweise 0,5 bis 65 Gew.-%, bezogen auf diese $SO_3$-haltige Schwefelsäure. Für die Berechnung der oben angegebenen Molmenge $H_2SO_4$ pro Mol Amin werden in einem solchen Falle die Molzahlen der Schwefelsäure und des $SO_3$ zusammengezählt. Bevorzugt wird Schwefelsäure mit 70 bis 100 Gew.-%, besonders bevorzugt mit 96 bis 100 Gew.-% $H_2SO_4$ und ganz besonders bevorzugt Monohydrat eingesetzt.

Erfindungsgemäß wird in Gegenwart eines Lösungsmittels gearbeitet.

Als Lösungsmittel seien beispielsweise alkyl- und/oder halogensubstituierte Aromaten als geeignet genannat. Hierunter seien beispielsweise ein Benzol, ein Naphthalin, Anthracen oder Diphenyl verstanden,

das bis zu 4 Alkylgruppen und/oder bis zu 4 Halogenatome, wie Fluor, Chlor oder Brom, als Substituenten tragen kann, wobei die Gesamtzahl der Substituenten naturgemäß durch die maximale Anzahl der zu substituierenden Positionen am aromatischen Grundkörper begrenzt ist. Als Alkylsubstituenten seien beispielsweise solche mit 1 bis 4, bevorzugt 1 bis 2, bevorzugt 1, C-Atome genannt, wie Methyl, Ethyl, Propyl, Isopropyl, Butyl oder Isobutyl. Zwei benachbarte Alkylsubstituenten können gemeinsam auch eine Alkylenkette mit 3 bis 5 C-Atomen bilden, wie Trimethylen, Tetramethylen oder Pentamethylen.

Beispiele für solche Lösungsmittel sind: Toluol, Xylol, Trimethylbenzol, Ethylbenzol, Methylnaphthalin, Tetrahydronaphthalin, Methylanthracen, Methyl-diphenyl, Fluorbenzol, Chlorbenzol, Brombenzol, 1,2-Dichlorbenzol, 1,3-Dichlorbenzol, 1,4-Dichlorbenzol, 1,2-Dibrombenzol, 1,3-Dibrombenzol, 1,4-Dibrombenzol, 1,2-, 1,3-, 1,4-Difluorbenzol, Trifluormethylbenzol, chlorierte Biphenyle, mono- und/oder polychloriertes Naphthalin, 1,2,4-Trichlorbenzol, 1,2,3-Trichlorbenzol, 1,3,5-Trichlorbenzol, 2-Chlor-toluol, 3-Chlor-toluol, 4-Chlor-toluol, 2-Bromtoluol, 3-Brom-toluol, 4-Brom-toluol, 2,6-Dichlortoluol, 2,4-Dichlortoluol, 2,5-Dichlortoluol, 2,3-Dichlortoluol, 3,4-Dichlortoluol. Solche Lösungsmittel können sowohl einzeln als auch im Gemisch angewendet werden, beispielsweise technische Dichlorbenzol-, Dichlortoluol- oder Trichlorbenzolgemische. Als Lösungsmittel können aber auch aliphatische Kohlenwasserstoffe, wie Petroleum, Kerosin oder Paraffine, wie Isododecan oder Decalin, sowie deren Gemische mit geeigneten Siedepunkten eingesetzt werden. Als Lösungsmittel werden bevorzugt alkyl- und/oder halogensubstituierte Benzole eingesetzt, bei denen selbstverständlich die Gesamtzahl der Substituenten auf maximal 6 begrenzt ist. Besonders bevorzugt werden durch 1 bis 3 Halogenatome substituierte Benzole eingesetzt, die daneben noch eine Methylgruppe tragen können. In ganz besonders bevorzugter Weise werden Dichlor- und/oder Trichlorbenzole und/oder -toluole eingesetzt; beispielhaft seien hierbei 1,2-Dichlorbenzol, 1,2,4-Trichlorbenzol und das technische Gemisch der Dichlortoluole genannt.

Das Lösungsmittel wird in einer Menge von 100 bis 2000 ml, bevorzugt 100 bis 500 ml, pro Mol Arylamin eingesetzt.

Das beim Zusammengeben des Arylamins und der Schwefelsäure, gegebenenfalls in einem der genannten Lösungsmittel, erhältliche Arylammoniumhydrogensulfat wird sodann erfindungsgemäß bei einer Temperatur von mindestens 140°C mindestens teilweise in Gegenwart von Wasser unter Druck zu der zugehörigen Amino-arylsulfonsäure umgesetzt.

Als Temperatur sei allgemein 140 bis 280°C, bevorzugt 150 bis 260°C, besonders bevorzugt 180 bis 250°C, genannt.

Das erfindungsgemäße Verfahren kann beispielsweise in Reaktoren, wie Autoklaven, Druckkesseln, Allphasenreaktoren oder Reaktoren vom Typ des Schaufeltrockners kontinuierlich oder diskontinuierlich durchgeführt werden.

Der erfindungsgemäß einzustellende Druck wird gewählt zwischen dem Partialdampfdruck des Lösungsmittels unter den Bedingungen des Verfahrens und dem maximal möglichen Druck des Reaktionssystems. Der maximal mögliche Druck des Reaktionssystems ist der Druck, der sich im geschlossenen Reaktor einstellt, und ist abhängig von der Reaktionstemperatur, der Art des Lösungsmittels und dem Grad der Umsetzung. Selbstverständlich ist es möglich, dem Reaktionssystem zusätzlich zu dem sich einstellenden Druck ein Inertgas wie Stickstoff oder Edelgase aufzudrücken.

Für den Druck sei beispielsweise ein Bereich von 1,1 bis 50 bar, bevorzugt 1,1 bis 20 bar, besonders bevorzugt 1,1 bis 10 bar, genannt.

Erfindungsgemäß wird die Umsetzung, des Arylammoniumhydrogensulfats zur zugehörigen Amino-arylsulfonsäure im Reaktionsabschnitt, in dem 70 bis 100% des gesamten Reaktionsumsatzes erzielt werden, in Gegenwart von Wasser durchgeführt. Selbstverständlich kann auch in anderen Reaktionsabschnitten in Gegenwart von Wasser gearbeitet werden. Als Wassermenge, die erfindungsgemäß während des Ablaufs des sogenannten Backverfahrens im Reaktor im genannten Reaktionsabschnitt anwesend ist, sei 0,02 bis 2 Mol, bevorzugt 0,02 bis 0,5 Mol, besonders bevorzugt 0,02 bis 0,2 Mol, Wasser pro Mol ursprünglich in das Reaktionsgemisch eingebrachtes Arylamin genannt. Dieses Wasser kann, zumindest teilweise, das beim Backverfahren entstehende Reaktionswasser sein. Es kann jedoch auch zusätzliches zum Ansatz gegebenes Wasser sein, beispielsweise dadurch, daß eine wasserhaltige Schwefelsäure eingesetzt wird. Im Reaktionsgemisch vorhandenes Wasser, dessen Menge die angegeenen Mengen übersteigt, ist zu Beginn des erfindungsgemäßen Verfahrens nicht schädlich. Solche überschüssigen Mengen Wasser werden jedoch im weiteren Verlauf der Reaktion des erfindungsgemäßen Verfahrens abdestilliert. Auch das erfindungsgemäß während der Reaktion anwesende Wasser wird vor Ende der Reaktion aus dem Reaktionsgemisch vollständig entfernt. Die Entfernung von Wasser während des Reaktionsablaufes des erfindungsgemäßen Verfahrens kann in kontinuierlicher oder diskontinuierlicher Weise erfolgen, so daß das Reaktionsgemisch gegen Ende der Reaktion nur noch eine Menge Wasser im unteren Teil des obengenannten Bereiches enthält. Dadurch kann das vollständige Entfernen des Wassers nahezu mit dem Ende der Reaktion zusammenfallen.

Das erfindungsgemäße Verfahren kann beispielsweise so durchgeführt werden, daß das Arylamin und die Schwefelsäure, gegebenenfalls mitverwendetes Lösungsmittel und gegebenenfalls im Rahmen der obigen Beschreibung zusätzlich mitverwendetes Wasser in beliebiger Reihenfolge in ein Druckgefäß gegeben werden. Der bei der exothermen Neutralisationsreaktion zwischen dem Arylamin und der Schwefelsäure auftretende Temperaturanstieg braucht hierbei nicht besonders berücksichtigt zu werden. Das Druckgefäß wird sodann verschlossen und auf eine Temperatur von mindestens 140°C gebracht. Aus

dem Reaktionsansatz wird nunmehr kontinuierlich oder absatzweise, gegebenenfalls nach einer kurzen Anlaufphase der Reaktion, Wasser nur in einer solchen Menge entfernt, daß stets Wasser im Rahmen der obigen Beschreibung im Reaktionsansatz verbleibt. Mit dem aus dem Druckreaktor herausdestillierenden Wasser kann gegebenenfalls auch etwas von dem mitverwendeten Lösungsmittel destillieren. Dieses herausdestillierte Lösungsmittel kann in einem Wasserabscheider vom Wasser getrennt werden und in geeigneter Weise in den Druckreaktor zurückgeführt werden. Durch eine geeignete Druckhaltung an der Desitllatentnahme kann sichergestellt werden, daß niemals das gesamte im Reaktionsgemisch vorhandene Wasser daraus entfernt wird. Es ist grundsätzlich auch möglich, die Kondensation und die Trennung von Wasser und mitdestilliertem Lösungsmittel unter dem im Reaktor herrschenden Druck durchzuführen. Hierbei kann evtl. mitdestilliertes Lösungsmittel in technisch einfacher Weise über einen unter dem Reaktionsdruck stehenden Wasserabscheider in den Reaktor zurückgeführt werden. Selbstverständlich ist es ebenso möglich, die Menge des erfindungsgemäß im Reaktionsgemisch erforderlichen Wassers durch kontinuierliche Zuspeisung zum Reaktionsgemisch, beispielsweise über eine Pumpe, aufrechtzuerhalten.

In einer weiteren Variante kann das Lösungsmittel auf die oben angegebene Temperatur vorgeheizt werden.

Hierzu können sodann die Ausgangskomponenten gemeinsam in Form einer Schmelze, Suspension oder Lösung oder einzeln simultan oder nacheinander zugegeben werden.

In einer anderen Variante des erfindungsgemäßen Verfahrens kann man auch nach Zusammengeben der Reaktionspartner sowie des Lösungsmittels und gegebenenfalls des zusätzlich zugegebenen Wassers dieses Gemisch bei Normaldruck und gleichzeitigem Abdestillieren von Wasser erhitzen, bis der Umsatz der Backung 70% des Gesamtumsatzes erreicht hat. Hierbei kann auch eine Temperatur erreicht werden, die bereits 140°C oder darüber beträgt. Erst danach wird der Reaktor geschlossen, und unter Anlegen des gewünschten Druckes, beispielsweise des Eigendruckes der Reaktionsmischung, wird weiter verfahren, wie oben bereits beschrieben.

Das nach vollständigem Umsatz erhaltene Reaktionsgemisch kann, gegebenenfalls nach Abkühlung auf unter 100°C, in verschiedener Weise aufgebracht werden. So ist es beispielsweise möglich, die im Lösungsmittel nicht oder nur wenig lösliche Amino-arylsulfonsäure durch Abfiltrieren oder Zentrifugieren vom Lösungsmittel zu trennen. Das Reaktionsgemisch kann jedoch auch einer alkalisch-wäßrigen Extraktion unterzogen werden, wobei Amino-arylsulfonsäure in Form ihres Salzes in die wäßrige Phase übergeht. Als alkalisch reagierende Stoffe für eine solche alkalisch-wäßrige Extraktion können beispielsweise Natriumhydroxid, Kaliumhydroxid, Calciumhydroxid, Magnesiumhydroxid, Natriumcarbonat, Kaliumcarbonat, Caliumcarbonat, Magnesiumcarbonat, Ammoniak oder aliphatische Amine eingesetzt werden. Vorzugsweise werden Natrium- oder Kaliumhydroxid eingesetzt. Der wäßrige Extrakt wird sodann zweckmäßigerweise durch Andestillieren von Spuren des Lösungsmittels befreit. Die so erhältliche klare, nahezu farblose Salzlösung der Aminoarylsulfonsäure kann im allgemeinen ohne jegliche weitere Reinigung eingesetzt werden. Gegebenenfalls kann diese wäßrige Phase jedoch auch mit geeigneten Adsorbentien, wie Aktivkohle oder polymeren organischen Adsorptionsmitteln, behandelt werden. Dieser wäßrige Extrakt kann jedoch auch durch Eindampfen auf das der verwendeten alkalischen Verbindung korrespondierende Salz der Aminoarylsulfonsäure aufgearbeitet werden. Weiterhin kann dieser Extrakt auch durch Ansäuern mit einer Mineralsäure, wie Salzsäure oder Schwefelsäure, behandelt werden, wobei die freie, sehr reine Aminoarylsulfonsäure ausfällt und beispielsweise durch Filtration gewonnen werden kann.

Mit Hilfe des erfindungsgemäßen Verfahrens kann eine sehr reine Amino-arylsulfonsäure in Ausbeuten über 95% hergestellt werden. Insbesondere ist deren Gehalt an unerwünschter Aminoaryldisulfonsäure, isomeren Aminoarylsulfonsäuren und gegebenenfalls nicht umgesetztem Amin sehr niedrig, im allgemeinen jeweils kleiner als 2,0 Gew.-%, bevorzugt kleiner als 0,5 Gew.-%, an den genannten Verbindungen. Die erfindungsgemäß erhältlichen Amino-arylsulfonsäuren stellen weiterhin ein äußerst helles Produkt dar, beispielsweise gemessen an der Farbzahl nach Hazen einer 20 gew.-%igen wäßrigen Natrium-amino-arylsulfonatlösung. Diese Hazen-Farbzahl ist beispielsweise bei erfindungsgemäß hergestellter p-Sulfanilsäure etwa 4, während sie bei nach dem Stand der Technik hergestellter Ware bei mindestens 13 liegt. Diese Tatsache ist beispielsweise für den Einsatz von Aminoarylsulfonsäuren zur Darstellung von optischen Aufhellern von großer Bedeutung.

Ein weiterer Vorteil des erfindungsgemäßen Verfahrens besteht darin, daß durch die Anwendung von Druck die Auswahl der in Frage kommenden Lösungsmittel sehr groß ist. Bei der Abnahme des Wassers aus dem Reaktionsgemisch unter Druck wird weiterhin nur ein äußerst geringer Anteil des Lösungsmittels im Vergleich zum Backen bei Normaldruck oder im Vakuum gleichzeitig abdestilliert, wodurch die Energiekosten des Verfahrens niedrig gehalten werden können.

Dieser letztere Vorteil der geringen mitzudestillierenden Lösungsmittelmenge im erfindungsgemäßen Verfahren soll beispielhaft an der folgenden tabellarischen Gegenüberstellung gezeigt werden. Alle Angaben beziehen sich auf die Backung von 2,5 Mol Aniliniumsulfat mit maximal 45 ml Reaktionswasser. Verglichen werden Backungen in o-Dichlorbenzol und in 1,2,4-Trichlorbenzol nach dem Stand der Technik (1 bar) und erfindungsgemäß (Angabe des typischen Überdruckes am Anfang und Ende der $H_2O$-Destillation) sowie die Menge des mitdestillierten Lösungsmittels (LM), das Volumenverhältnis $H_2O$:LM und die Temperatur.

| LM | ml dest. | $H_2O$:LM | Druck (bar) | Temp. (°C) |
|---|---|---|---|---|
| Dichlorb. | 6750 | 1:150 | 1 | 180 |
| Dichlorb. | 50 | 1:1,1 | 2,4 bis 1,6 | 200 |
| Trichlorb. | 850 | 1:19 | 1 | 190 bis 210 |
| Trichlorb. | 29 | 1:0,64 | 7,9 bis 1,3 | 240 |

Entsprechend den Vorstellungen, die man sich bisher vom Ablauf und der Durchführung des sogenannten Backverfahrens gemacht hatte, schien es notwendig zu sein, das bei der Umwandlung des Arylammoniumhydrogensulfates in die korrespondierende Amino-arylsulfonsäure entstehende Reaktionswasser vollständig und möglichst umgehend aus dem Reaktionsgemisch zu entfernen, um die Reaktion in Richtung der Amino-arylsulfonsäure voranzubringen. Überraschenderweise wurde gefunden, daß entgegen dieser vorherrschenden Meinung das Backverfahren vorteilhaft in Gegenwart von Wasser durchgeführt werden kann, wobei hellere Produkte entstehen, die weniger unerwünschte Nebenprodukte enthalten.

Die im erfindungsgemäßen Verfahren erhältlichen Aminoarylsulfonsäuren sind wertvolle Zwischenprodukte für die Herstellung von Pharmazeutika, Schaumstoffen, optischen Aufhellern, Netzmitteln, synthetischen Beizmitteln, Gerbstoffen, Reservierungsmitteln, Insektiziden, Appreturmitteln, Weichmachern und polymeren Verdickungsmitteln (Ullmanns Enzyklopädie der technischen Chemie, 3. Auflage, Band 16, Seite 561, Verlag Urban und Schwarzenberg, München/Berlin 1965).

Beispiel 1 (zum Vergleich)

(nach B. I. Kissin, E. N. Kurakin, Khim. Prom. *41*, 104 (1965))

186,2 g (2 Mol) Anilin werden in 500 ml 1,2-Dichlorbenzol vorgelegt und 208,3 g (2,04 Mol) 96 %ige Schwefelsäure zugetropft. Es wird 8 Stunden am Wasserabscheider auf Siedetemperatur erhitzt, wobei pro Stunde eine Destillatmenge von 700 ml übergeht, abgekühlt und abgesaugt. Der Niederschlag wiegt nach dem Trocknen 338,5 g und enthält 86,1% p-Sulanilsäure (84,2% der theoretischen Ausbeute) und 11,2% Anilin-2,4-Disulfonsäure (7,5%, bezogen auf Anilin).

Beispiel 2

651,7 g (7,0 Mol) Anilin werden in 1400 ml 1,2-Dichlorbenzol in einem 4-1-Emaille-Rührautoklaven vorgelegt und mit 686,7 g (7,0 Mol) Monohydrat versetzt. Der Autoklav wird geschlossen und auf 200°C Innentemperatur erhitzt. In 2,75 Stunden werden über ein Ventil 122 ml Wasser mit 140 ml Lösungsmittel entfernt, wobei der anfängliche Druck von 2,4 bar auf 1,6 bar absinkt. Die Suspension wird nach dem Abkühlen auf 120°C heiß abgesaugt und ergibt nach dem Trocknen 1203 g Rückstand. Der Gehalt an p-Sulfanilsäure in diesem Rückstand beträgt 98,0 Gew.-% und der an Anilin-2,4-disulfonsäure 0,5 Gew.-%. Daraus errechnet sich eine Ausbeute an p-Sulanilsäure von 97,2% der theoretischen Ausbeute. Die Farbzahl einer 20 %igen wäßrigen Natriumsalz-Lösung nach Hazen ist 3.

Beispiel 3 (zum Vergleich)

(nach B.I. Kissin, UdSSR-Patent Nr. 66 7550).

150 g (1,61 Mol) Anilin werden in einem Gemisch von 310 ml 1,2-Dichlorbenzol und 62 ml 1,2,4-Trichlorbenzol vorgelegt. Innerhalb von 60 Minuten werden 160 g (1,63 Mol) Monohydrat zugetropft, wobei die Temperatur auf 123°C ansteigt. Es wird zum Sieden (172°C) erhitzt und im Laufe von 8 Stunden 28 ml Wasser und 186 ml Lösungsmittelgemisch abdestilliert, wobei die Temperatur bis auf 182°C ansteigt, und anschließend weitere 6 Stunden am Wasserabscheider erhitzt. Man erhält eine dunkle, dickflüssige Suspension. Im Wasserstrahlvakuum wird restliches Lösungsmittel abdestilliert (154 ml), der Rückstand mit 450 ml Wasser versetzt und mit 120 ml 40 %iger NaOH neutralisiert. Die Lösung wird von schwarzen Rückständen abgesaugt, anschließend 1 Stunde mit Wasserdampf destilliert und zur Trockne eingeengt. Der graue Rückstand wiegt nach dem Trocknen 295 g und enthält 84,9 Gew.-% p-Sulfanilsäure (89,8% Ausbeute, bezogen auf Anilin bzw. 88,7%, bezogen auf $H_2SO_4$ und 2,2 Gew.-% Anilin-2,4-disulfonsäure). Farbzahl einer 20 %igen wäßrigen Natriumsalz-Lösung nach Hazen = 13.

Beispiel 4

470,1 g (5,05 Mol) Anilin werden in 1000 ml 1,2,4-Trichlorbenzol in einem Rührautoklaven vorgelegt und 490 g (5,0 Mol) Monohydrat in 15 Minuten zugetropft. Man heizt den geschlossenen Autoklaven auf 240°C Innentemperatur und entfernt das Reaktionswasser (90 ml) mit geringen Lösungsmittelmengen (58 ml) durch stetiges Entspannen und Kondensieren im Verlaufe von 40 Minuten bei einem Druck von anfänglich 7,8 bar, der nach dem Öffnen des Ventils auf 3,2 bar absinkt und im Verlaufe der Reaktion weiter auf einen Druck von 1,3 bar absinkt. Man rührt noch 50 Minuten bei dieser Temperatur und dem Druck von

1,3 bar nach. Dann werden 3100 ml Wasser und 374 ml 50 %ige Kaliumhydroxidlösung zugegeben, die Phasen getrennt und von der wäßrigen Phase 460 ml abdestilliert. Die wäßrige Kaliumsalzlösung wiegt sodann 4218,5 g und enthält 20,1 Gew.-% p-Sulfanilsäure (98% der theoretischen Ausbeute). Der Gehalt an Anilin-2,4-disulfonsäure beträgt 0,054 Gew.-%. Die Farbzahl einer 20 %igen Natriumsalz-Lösung nach Hazen beträgt 4.

Beispiel 5

470,1 g (5,05 Mol) Anilin werden in 1000 ml Dichlortoluol in einem Rührautoklaven vorgelegt und 490 g (5,0 Mol) Monohydrat in 30 Minuten zugetropft. Die Temperatur steigt auf etwa 150°C an. Es wird auf 210°C geheizt und bei dieser Temperatur und bei einem Anfangsdruck von 2,1 bar das Reaktionswasser mit einem geringen Anteil Lösungsmittel stetig entnommen. Nach etwa 60 Minuten ist die Entfernung des Reaktionswassers beendet, und es wird noch 30 Minuten bei einem Druck von 1,2 bar nachgerührt. Der Reaktionsansatz wird wie in Beispiel 4 aufgearbeitet. Man erhält 1014,2 Kaliumsalz der p-Sulfanilsäure (98,4 %ig). Der Gehalt an p-Sulfanilsäure beträgt 80,7% (94,5% der theoretischen Ausbeute), der Gehalt an Anilin-2,4-disulfonsäure beträgt 1,0%.

Beispiel 6

305,6 (3,28 Mol) Anilin werden in 650 ml 1,2-Dichlorbenzol in einem 2-1-Glasautoklaven vorgelegt. Bei Normaldruck werden 318,8 g (3,25 Mol) Monohydrat in 10 Minuten unter Rühren zugetropft, wobei die Temperatur auf 154°C ansteigt. Der Autoklav wird mit einem Druckhalteventil, das bei einem Differenzdruck von 1,3 bar automatisch öffnet, ausgerüstet und auf 200°C Innentemperatur geheizt. Über das Druckhalteventil wird entstehendes Reaktionswasser mit geringen Lösungsmittelmengen abdestilliert. Nach 2 Stunden sind etwa 56 ml Wasser (von 58,5 ml theoretisch möglichen) zusammen mit 80 ml Lösungsmittel abdestilliert. Es wird noch 1 Stunde nachgerührt, wobei der Autoklav geschlossen ist. Die entstandene Suspension wird abgesaugt und der Filterrückstand getrocknet. Die isolierte p-Sulfanilsäure hat ein Gewicht von 556,3 g und einen Gehalt von 99,5 Gew.-%; der Gehalt an Anilin-2,4-disulfonsäure liegt bei 0,4 Gew.-%. Die Ausbeute beträgt 98,3%, bezogen auf Schwefelsäure.

Beispiel 7

214 g (2,0 Mol) o-Toluidin werden in 1000 ml 1,2-Dichlorbenzol in einem 3-1-Emailleautoklaven vorgelegt und bei Normaldruck mit 196,2 g (2,0 Mol) Monohydrat versetzt. Der Autoklav wird geschlossen und auf 200°C Innentemperatur erhitzt, wobei der Druck auf 3,1 bar ansteigt. Über ein Ventil wird das Reaktionswasser zusammen mit geringen Lösungsmittelmengen abdestilliert, wobei der Druck auf 1,1 bar absinkt. Nach 1,5 Stunden ist das Reaktionswasser (34 ml) gemeinsam mit 40 ml 1,2-Dichlorbenzol abdestilliert. Die Suspension wird abgesaugt, der Filterrückstand wiegt nach dem Trocknen 371,6 g. Der Gehalt an 4-Amino-3-methyl-benzolsulfonsäure beträgt 99,6 Gew-%, was einer Ausbeute von 99% der theoretischen Ausbeute entspricht. Der Gehalt an nicht-umgesetztem Toluidin liegt bei 0,2 Gew.-%.

Beispiel 8

286,4 g (2,0 Mol) α-Naphthylamin werden in einem 3-1-Emaille-Rührautoklaven in 1000 ml 1,2-Dichlorbenzol vorgelegt und mit 196,2 g (2,0 Mol) Monohydrat versetzt. Nach 15 Minuten ist die Zugabe beendet. Der Autoklav wird verschlossen und auf 190°C erhitzt, wobei der Druck auf 3 bar ansteigt. nach 2,5 Stunden Reaktionszeit sind 33 ml Reaktionswasser zusammen mit 60 ml Lösungsmittel über ein Ventil herausdestilliert. Die körnige Suspension wird mit wäßriger Natronlauge extrahiert, wobei nicht-umgesetztes α-Naphthylamin in der organischen Phase bleibt und im nächsten Ansatz wieder verwendet werden kann. Die wäßrige Phase wird zur Trockne eingeengt, wobei 465,6 g p-Naphthionsäure (90,1 %ig) zurückbleiben, was einer Ausbeute von 92,0% der theoretischen Ausbeute entspricht.

Beispiel 9

255,2 g (2,0 Mol p-Chloranilin werden in einem 3-1-Emaille-Rührautoklaven in 1000 ml 1,2-Dichlorbenzol vorgelegt und bei Normaldruck mit 196,2 g (2,0 Mol) Monohydrat versetzt. Bei geöffnetem Ventil wird der Autoklav aufgeheizt. Bei beginnender Destillation wird das Ventil so weit geschlossen, daß die Destillatmenge gering gehalten wird und die gewünschte Reaktionstemperatur von 200°C erreicht wird. Bei einem maximalen Druck von 1,6 bar werden nunmehr stetig 32 ml Reaktionswasser gemeinsam mit 90 ml Lösungsmittel abdestilliert. Die Suspension wird heiß abgesaugt. Der Niederschlag wiegt nach dem Trocknen 412,9 g und enthält 98 Gew.-% 2-Amino-5-chlor-benzolsulfonsäure, was einer Ausbeute von 97,5% der theoretischen Ausbeute entspricht.

Beispiel 10

255,2 g (2,0 Mol) o-Chloranilin werden in 1000 ml 1,2-Dichlorbenzol in einem Rührautoklaven vorgelegt und mit 196,2 g (2,0 Mol) Monohydrat versetzt. Man heizt auf 200°C und destilliert in 1,5 Stunden 33 ml Reaktionswasser über ein 2 mm-Ventil ab, wobei nur 40 ml Lösungsmittel mitdestilliert werden. Der anfängliche Druck von 2,4 bar sinkt gegen Ende der Reaktion auf 1,6 bar. Die Suspension wird abgesaugt und der Niederschlag getrocknet. Der Rückstand wiegt 417,4 g und hat einen Gehalt von 98,5 Gew.-% 4-Amino-3-Chlorbenzolsulfonsäure und 0,1 Gew.-% o-Chloranilin. Die Ausbeute beträgt 99,0% der theoretischen Ausbeute.

Beispiel 11

324,2 g (2,0 Mol) 3,4-Dichloranilin werden in 1000 ml 1,2-Dichlorbenzol in 3-1-Emaille-Rührautoklaven vorgelegt. Innerhalb von 10 Minuten werden 196,2 g (2,0 Mol) Monohydrat zugetropft und der gesamte Ansatz verschlossen auf 205°C erhitzt. Über ein Ventil werden in 1 Stunde 50 Minuten 33 ml Reaktionswasser mit nur 50 ml Lösungsmittel abdestilliert, wobei der Druck von 2,5 bar auf 1,2 bar abfällt. Der abgesaugte Niederschlag wiegt nach dem Trocknen 477,6 g und enthält 96,9 Gew.-% 2-Amino-4,5-dichlor-benzolsulfonsäure; dies entspricht 95,6% der theoretischen Ausbeute.

Beispiel 12

283,2 g (2,0 Mol) 3-Chlor-4-methylanilin werden in 1000 ml 1,2-Dichlorbenzol in einem 3-1-Emaille-Rührautoklaven vorgelegt und in 7 Minuten mit 196,2 g (2,0 Mol) Monohydrat versetzt. Der geschlossene Autoklav wird auf 200°C erhitzt, wobei sich ein Druck von 2,8 bar einstellt. Sodann werden in 1 Stunde 25 Minuten 35 ml Reaktionswasser zusammen mit 80 ml Lösungsmittel abdestilliert, wobei der Druck bis auf 1,3 bar abfällt. Nach dem Absaugen und Trocknen bleiben 440,8 g 2-amino-4-chlor-5-methyl-benzolsulfonsäure mit einem Gehalt von 99,5 Gew.-% zurück. Dies entspricht 99,0% der theoretischen Ausbeute.

Beispiel 13

283,2 g (2,0 Mol) 4-Chlor-3-methylanilin werden, wie in Beispiel 12 beschrieben, mit 196,2 g (2,0 Mol) Monohydrat umgesetzt. Nach 1,5-stündiger Destillation über ein 2 mm-Ventil werden neben 34 ml Reaktionswasser noch 130 ml Lösungsmittel erhalten. Der Druck fällt hierbei von 2,5 bar auf 1,3 bar ab. Der Gehalt der trockenen 2-Amino-5-chlor-4-methyl-benzolsulfonsäure (438,5 g) beträgt 99,0%, was einer Ausbeute von 98,0% der theoretischen Ausbeute entspricht.

**Patentansprüche**

1. Verfahren zur Herstellung von Amino-arylsulfonsäuren durch Umsetzung von Arylaminen mit Schwefelsäure in Gegenwart eines Lösungsmittels nach dem sogenannten Backverfahren bei einer Temperatur von 140°C—280°C, dadurch gekennzeichnet, daß bei der Umsetzung im Reaktionsabschnitt, in dem 70—100% des gesamten Reaktionsumsatzes erzielt werden, Wasser nur in einer solchen Menge entfernt wird, daß stets 0,02—2 Mol Wasser pro Mol ursprünglich in das Reaktionsgemisch eingebrachten Amins im Reaktionsansatz verbleibt, und die Umsetzung im Druckbereich zwischen dem Partialdampfdruck des Lösungsmittels unter den Bedingungen des Verfahrens und dem max. möglichen Druck des Reaktionssystems durchgeführt wird, wobei man unter einem Druck von 1,1—50 bar arbeitet.

2. Verfahren nach Anspruch 1 dadurch gekennzeichnet, daß Arylamine der allgemeinen Formel

$$Ar^1—N(R^1,R^2)$$

eingesetzt werden.
in der
$R^1$ und $R^2$ unabhängig voneinander Wasserstoff, Alkyl, Aralkyl oder Aryl bedeuten oder beide gemeinsam mit dem N-Atom, das sie substituieren, einen Stickstoffheterocyclus bilden und $Ar^1$ das gegebenenfalls substituierte Benzol-, Naphthalin-, Anthracen-, Naphthochinon- oder Anthrachinongerüst oder das Gerüst eines aromatischen Heterocyclus darstellt.

3. Verfahren nach Ansprüchen 1 und 2, dadurch gekennzeichnet, daß Arylamine der allgemeinen Formel

$$Ar^2—N(R^1,R^2)$$

eingesetzt werden,
in der
$R^1$ und $R^2$ die in Anspruch 2 gegebene Bedeutung haben und
$Ar^2$ das Benzol- oder Naphthalingerüst darstellt.

4. Verfahren nach Ansprüchen 1 bis 3, dadurch gekennzeichnet daß Arylamine der allgemeinen Formel

$$Ar^2—NH_2$$

eingesetzt werden,
in der
$Ar^2$ die in Anspruch 3 gegebene Bedeutung hat.

5. Verfahren nach Ansprüchen 1 bis 4, dadurch gekennzeichnet, daß das Verfahren in Gegenwart von 0,02 bis 0,5 Mol Wasser pro Mol ursprünglich in das Reaktionsgemisch eingebrachten Amins durchgeführt wird.

**Revendications**

1. Procédé pour la fabrication d'acides aminoarylsulfoniques par réaction d'arylamines avec l'acide sulfurique en présence d'un solvant selon le procédé dit de cuisson à une température de 140—280°C, caractérisé en ce que dans la partie de la réaction dans laquelle on atteint 70—100% de la conversion totale,

on sépare seulement une quantité d'eau telle qu'il reste toujours dans le mélange de réaction 0,02—2 mol d'eau par mol d'amine introduite initialement dans le mélange de réaction, et la réaction est mise en oeuvre dans l'intervalle de pressions de la pression de vapeur partielle du solvant dans les conditions du procédé à la pression maximale possible du système de réaction, la pression opératoire étant de 1,1—50 bar.

2. Procédé selon la revendication 1, caractérisé en ce que l'on utilise des arylamines de formule générale:

$$Ar^1—N(R^1,R^2)$$

dans laquelle

$R^1$ et $R^2$ représentent, indépendamment l'un de l'autre, l'hydrogène, des groupes alkyle, aralkyle ou aryle ou forment ensemble et avec l'atome d'azote auquel ils sont liés un hétérocycle azoté et

$Ar^1$ représente le squelette du benzène, du naphtalène, de l'anthracène, de la naphtoquinone ou de l'anthraquinone éventuellement substitué ou le squelette d'un hétérocycle aromatique.

3. Procédé selon les revendications 1 et 2, caractérisé en ce qu'on utilise des arylamines de formule générale:

$$Ar^2—N(R^1,R^2)$$

dans laquelle

$R^1$ et $R^2$ ont les significations indiquées dans la revendication 2, et

$Ar^2$ représente le squelette du benzène ou du napthalène.

4. Procédé selon les revendications 1 à 3, caractérisé en ce que l'on utilise des arylamines de formule générale:

$$Ar^2—NH_2$$

dans laquelle

$Ar^2$ a la signification indiquée dans la revendication 3.

5. Procédé selon les revendications 1 à 4, caractérisé en ce que le procédé est mis en oeuvre en présence de 0,02—0,5 mol d'eau par mol d'amine initialement introduite dans le mélange de réaction.

**Claims**

1. Process for the preparation of amino-arylsulphonic acids by reacting arylamines with sulphuric acid in the presence of a solvent by the so-called baking process at a temperature of 140°C—280°C, characterized in that during the reaction, in the reaction stage in which 70—100% of the overall reaction conversion is achieved, water is removed only at a rate such that 0.02—2 mol of water per mol of amine originally introduced into the reaction mixture always remains in the reaction batch, and the reaction is carried out in the pressure range between the partial vapour pressure of the solvent under the process conditions and the maximum possible pressure of the reaction system, the operating pressure being 1.1—50 bar.

2. Process according to Claim 1, characterized in that arylamines of the general formula

$$Ar^1—N(R^1,R^2)$$

are used,

in which

$R^1$ and $R^2$, independently of one another, represent hydrogen, alkyl, aralkyl or aryl or these two radicals form, together with the N atom of which they are substituents, a nitrogen heterocycle and $Ar^1$ represents the optionally substituted benzene, naphthalene, anthracene, naphthoquinone or anthraquinone skeleton or the skeleton of an aromatic heterocycle.

3. Process according to Claims 1 and 2, characterized in that arylamines of the general formula

$$Ar^2—N(R^1,R^2)$$

are used,

in which

$R^1$ and $R^2$ have the meaning given in Claim 2 and $Ar^2$ represents the benzene or naphthalene skeleton.

4. Proces according to Claims 1 to 3, characterized in that arylamines of the general formula

$$Ar^2—NH_2$$

are used,

in which

$Ar^2$ has the meaning given in Claim 3.

5. Process according to Claims 1 to 4, characterized in that the process is carried out in the presence of 0.02 to 0.5 mol of water per mol of amine originally introduced into the reaction mixture.